# EUROPÄISCHE PATENTSCHRIFT

(11) **EP 3 870 130 B1**
(45) Veröffentlichungstag und Bekanntmachung des Hinweises auf die Patenterteilung: **19.06.2024**
(21) Anmeldenummer: 19808681.1
(22) Anmeldetag: 16.10.2019
(51) Int. Cl.: A61H 7/00, A61M 21/00

(54) **VORRICHTUNG ZUR ERHÖHUNG DES WOHLBEFINDENS VON LEBEWESEN**
DEVICE FOR INCREASING THE WELL-BEING OF LIVING BEINGS
DISPOSITIF AUGMENTER LE BIEN-ÊTRE D'ÊTRES VIVANTS

(30) Priorität: 26.10.2018 DE 102018126800; 12.11.2018 DE 102018128168
(43) Veröffentlichungstag der Anmeldung: 01.09.2021
(73) Patentinhaber: Casada Group GmbH, 33104 Paderborn (DE)
(72) Erfinder: RADTKE, Jakob, 33106 Paderborn (DE)
(74) Vertreter: Ostermann, Thomas
(86) Internationale Anmeldenummer: PCT/DE2019/100896
(87) Internationale Veröffentlichungsnummer: WO 2020/083431

(56) Entgegenhaltungen:
- EP-A1- 0 422 253
- WO-A1-87/05497
- WO-A1-90/04379
- US-A- 4 834 701

## Beschreibung

Die Erfindung betrifft eine Vorrichtung zur Erhöhung des Wohlbefindens von Lebewesen mit einem Gehirnstimulator zur Erzeugung eines Stimulationssignals im Infraschallbereich, mittels dessen ein Gehirns des Lebewesens stimulierbar ist.

Aus der EP 2 069 001 A1 ist eine Vorrichtung zur Förderung eines erholsamen Schlafs bekannt. Hierzu wird ein Gehirnstimulator eingesetzt, der ein Stimulationssignal im Infraschallbereich erzeugt, welches über an einen Kopf des Menschen angebrachte Elektroden in das Gehirn desselben induziert wird. Es werden an dem Gehirn EEG-Signale detektiert, die in einem Frequenzbereich von 0,5 Hz bis 4 Hz konzentriert sind. Alternativ kann das Stimulationssignal auch über einen Kopfhörer in das Gehirn induziert werden. Die bekannte Vorrichtung ist auf die Anwendung von Schlafstörungen beschränkt. Wünschenswert ist es, Stress auch zur Nichtschlafenszeiten abzubauen, um das psychische und physische Wohlbefinden eines Lebewesens zu verbessern.

Aus der US 4 834 701 A ist eine Vorrichtung zur Erzeugung einer verringerten Frequenz von Gehirnwellen bekannt. Die Vorrichtung erzeugt unterschiedliche niederfrequente Signale. Eine zeitliche Änderung der Frequenz ist nicht vorgesehen.

Aus der WO 87/05497 A1 ist eine Vorrichtung zur Erhöhung des Wohlbefindens von Lebewesen mit einem Gehirnstimulator bekannt. Mittels des Gehirnstimulators wird ein Stimulationssignal im Infraschallbereich unter Bildung von Vibrationen erzeugt, so dass es als Massagesignal auf Lebewesen einen therapeutischen Effekt erzielen kann.

Aufgabe der vorliegenden Erfindung ist es daher, eine Vorrichtung zur Erhöhung des Wohlbefindens von Lebewesen derart anzugeben, dass effektiv der Grad des Entspannungszustandes erhöht bzw. der Entspannungszustand schneller eingenommen werden kann.

Zur Lösung dieser Aufgabe weist die Erfindung die Merkmale des Patentanspruchs 1 auf.

Der besondere Vorteil der Erfindung besteht darin, dass durch die kombinierte Anwendung von Gehirnstimulation einerseits und Massage von Körperpartien des Lebewesens andererseits die Entspannungswirksamkeit wesentlich verbessert werden kann. Untersuchungen haben ergeben, dass die Effektivität der Entspannung hierdurch bis zu 80 % erhöht werden kann. Grundgedanke der Erfindung ist es, zum einen durch Stimulation des Gehirns über die Psyche und zum anderen durch Massage physisch auf das Lebewesen derart einzuwirken, dass durch ihre kombinierte Anwendung sich ein verbesserter Entspannungszustand beim Lebewesen erreichen lässt, der über die summarische Wirksamkeit aus Gehirnstimulation und Massage weit hinausgeht. Studien haben gezeigt, dass sich der gewünschte Entspannungszustand signifikant schneller und dauerhafter einstellt, als wenn man die Gehirnstimulation und die Massage zeitversetzt vornimmt.

Nach der Erfindung wird der Gehirnstimulator derart angesteuert, dass in einem ersten Zeitintervall die Schwebungsfrequenz in einem Bereich zwischen 14 Hz bis 30 Hz verringert wird in einen Bereich von 7 Hz bis 14 Hz oder 3 Hz bis 7 Hz. Das Lebewesen befindet sich hierbei in einer Entspannungsphase. In einem nachfolgenden zweiten Zeitintervall befindet sich das Lebewesen in einer Anwendungsphase, in der die Schwebungsfrequenz ihren Tiefpunkt erreicht. In einem dritten Zeitintervall schließt sich eine Regenerationsphase an, in der die Schwebungsfrequenz wieder ansteigt im Bereich der ursprünglichen Frequenz. Wenn das Entspannungsprogramm zum Einschlafen genutzt werden soll, kann in dem dritten Zeitintervall niedrige Stimulationsfrequenz erzeugt werden. Das erste Zeitintervall, das zweite Zeitintervall und das dritte Zeitintervall können in einem Bereich zwischen 5 min und 10 min liegen.

Nach der Erfindung ist eine Ansteuereinrichtung vorgesehen, mittels derer ein Stimulationsansteuersignal zur Ansteuerung des Gehirnstimulators und ein Massageansteuersignal zur Ansteuerung der Massageeinrichtung erzeugbar ist. Diese Ansteuereinrichtung ist vorzugsweise in einem mobilen Datengerät, insbesondere in einem Mobiltelefon, integriert, so dass der Nutzer mittels einer Entspannungsapplikation bedienungsfreundlich auf die beiden Geräte, nämlich Gehirnstimulator und Massageeinrichtung, einwirken kann.

Nach einer bevorzugten Ausführungsform der Erfindung weist die Ansteuereinrichtung mehrere Entspannungsprogramme auf, so dass der Nutzer in Abhängigkeit von seinem aktuellen Zustand unterschiedlich intensive und/oder lange Entspannungsprogramme selbst wählen kann, um am Ende des Vorgangs den gewünschten Entspannungszustand einzunehmen.

Nach einer Weiterbildung der Erfindung ist das von dem Gehirnstimulator abgegebene Stimulationssignal als eine binaurale Schwebung mit einer vorzugsweise pulsierenden Schwebungsfrequenz ausgebildet, wobei unterschiedliche Trägerfrequenzen an unterschiedliche Ohren des Lebewesens induziert werden. Die Differenz der Trägerfrequenzen liegt im niedrigfrequenten Infraschallbereich. Der Hörapparat des Lebewesens nimmt die unterschiedlichen Schwingungen des Stimulationssignales nicht wahr. Stattdessen wird der Interferenzprozess in das Gehirn verlagert, wobei das Ohr des Lebewesens umgangen wird. Vorzugsweise erfolgt die Synchronisation von mindestens zwei Rhythmuszyklen des Stimulationssignals.

Nach einer Weiterbildung der Erfindung wird dem Stimulationssignal ein Musiksignal überlagert, so dass durch die Musik eine zusätzlich entspannende Wirkung auf das Lebewesen ausgeübt wird.

Nach einer Weiterbildung der Erfindung ist die Massageeinrichtung als ein Massagesessel mit Mitteln zur Rückenmassierung ausgebildet. Durch eine feinfühlige Rückenmassage kann die sanfte Regeneration unterversorgter Bandscheiben eingeleitet werden. Das Lösen von seelischen und körperlichen Blockaden wird gefördert.

Ausführungsbeispiele der Erfindung werden nachfolgend anhand der Zeichnungen näher erläutert.

Es zeigen:

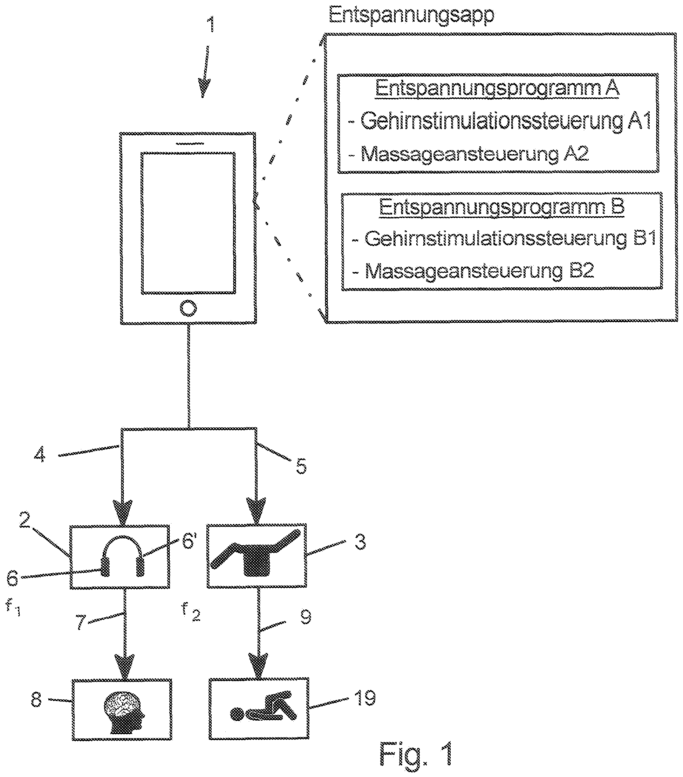

- Figur 1: eine schematische Darstellung einer erfindungsgemäßen Vorrichtung,
- Figur 2: ein Frequenz-Zeitdiagramm entsprechend einem ersten Entspannungsprogramm und
- Figur 3: ein Frequenz-Zeitdiagramm entsprechend einem zweiten Entspannungsprogramm.

Eine Vorrichtung zur psychophysiologischen Entspannung von Lebewesen wird vorzugsweise für Menschen eingesetzt. Alternativ kann die erfindungsgemäße Vorrichtung auch den Entspannungszustand von Tieren, beispielsweise bei dem Transport von Pferden oder Hunden, verbessern, so dass keine Medikamente eingenommen werden müssen.

Die erfindungsgemäße Vorrichtung sieht eine Ansteuereinrichtung 1 zur Ansteuerung eines Gehirnstimulators 2 und einer Massageeinrichtung 3 vor. Die Ansteuereinrichtung 1 ist als ein mobiles Datengerät, vorzugsweise als Mobiltelefon (Smartphone), ausgebildet, welches Bedien-, Anzeige- und Steuermittel in der üblichen Weise aufweist. In dem mobilen Datengerät 1 ist eine Entspannungsapplikation implementiert, mittels derer eine Bedienperson handhabungstechnisch einfach auf die Geräte 2, 3 einwirken kann. Das mobile Datengerät 1 ist über eine drahtlose Kommunikationsschnittstelle, beispielsweise Bluetooth oder WLAN, mit dem Gehirnstimulator 2 und der Masseeinrichtung 3 gekoppelt.

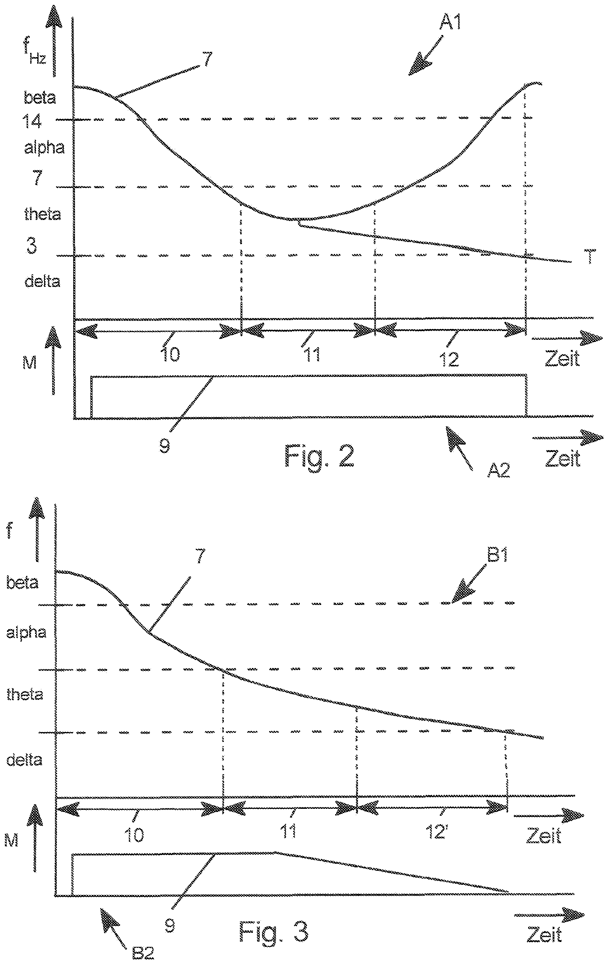

Die Entspannungsapplikation kann mehrere Entspannungsprogramme aufweisen, die die Bedienperson wahlweise starten und durchlaufen kann. Beispielsweise kann die Entspannungsapplikation ein Entspannungsprogramm A aufweisen, dessen Gehirnstimulationsansteuerung A1 und Massageansteuerung A2 in Figur 2 dargestellt ist. Beispielsweise kann die Entspannungsapplikation ein weiteres Entspannungsprogramm B aufweisen, dessen Gehirnstimulationsansteuerung B1 und Massageansteuerung B2 in Figur 3 dargestellt ist. Vorzugsweise kann die Entspannungsapplikation weitere Entspannungsprogramme aufweisen.

Mit Starten des Entspannungsprogramms A wird ein Stimulationsansteuersignal 4 an den Gehirnstimulator 2 und ein Massageansteuersignal 5 an die Massageeinrichtung 3 übermittelt. Der Gehirnstimulator 2 kann beispielsweise als ein drahtlos oder drahtgebundener betriebener Kopfhörer ausgebildet sein mit zwei Hörkapseln 6, 6', die jeweils auf ein Ohr des Lebewesens gesetzt werden.

Die Massageeinrichtung 3 ist vorzugsweise als ein Massagesessel ausgebildet, der Massagemittel zur Massage insbesondere eines Rückens des Lebewesens vorsieht.

Der Gehirnstimulator 2 erlangt ein Stimulationssignal 7, das unter Umgehung des Ohres direkt auf ein Gehirn 8 des Lebewesens wirkt. Die Massageeinrichtung 3 wirkt mit einem Massagesignal 9 direkt auf Körperpartien 19 des Lebewesens bzw. einer Person, die vorzugsweise die Ansteuereinrichtung 1 bedient.

Das Stimulationssteuersignal 4 ist derart ausgebildet, dass an der einen Hörkapsel 6 ein Stimulationssignal im Infraschallbereich einer ersten Frequenz f1 und an der zweiten Hörkapsel 6' ein unterschiedliches Stimulationssignal einer Frequenz f2 im Infraschallbereich erzeugt wird. Unter Bildung einer Schwebung bildet sich eine Schwebungsfrequenz, unter der das Stimulationssignal 7 als Differenz zwischen der ersten Frequenz f1 und der zweiten Frequenz f2 an das Gehirn 8 induziert wird. Vorzugsweise handelt es sich um ein pulsierendes Stimulationssignal 7. Im vorliegenden Ausführungsbeispiel kann die eine Trägerfrequenz f1 114 Hz und die zweite Trägerfrequenz f2 124 Hz betragen, so dass sich eine Schwebungsfrequenz von 10 Hz für das Stimulationssignal 7 ergibt. Vorzugsweise ergibt sich eine rhythmische Stimulation innerhalb des Gehirns, wobei sich die Frequenzen des Stimulationssignals 7 mittels eines Elektroenzephalogramms (EEG) aufzeichnen lassen. Die Schwebungsfrequenz kann in einem Bereich zwischen 1 Hz und 30 Hz liegen. Vorzugsweise ist sie über den Entspannungsvorgang konstant. Alternativ kann die Schwebungsfrequenz innerhalb eines Entspannungsprogramms auch variieren.

Nach dem Entspannungsprogramm A gemäß Figur 2 wird mit Starten des Entspannungsprogramms A die Gehirnstimulatoransteuerung A1 und die Massageansteuerung A2 vorzugsweise gleichzeitig eingeschaltet. In einem ersten Zeitintervall 10 (Entspannungsphase) beträgt die Stimulationsfrequenz 14 Hz bis 30 Hz. Beispielsweise kann sie aus einem Beta-Frequenzband (14 Hz bis 30 Hz) kontinuierlich unter Durchlaufen eines Alpha-Frequenzbandes (7 Hz bis 14 Hz) bis in einen Theta-Frequenzband (3 Hz bis 7 Hz) absinken. Nach Ablauf von beispielsweise 8 min beginnt dann das zweite Zeitintervall 11 (Anwendungsphase), in dem sich die Stimulationsfrequenz durchgehend in dem Theta-Frequenzband befindet. Innerhalb dieser Anwendungsphase liegt ein Tiefpunkt T einer Verlaufskurve des Stimulationssignals 7. In der Anwendungsphase befindet sich die Person in einem entspannten bzw. schläfrigen Zustand. Nach Ablauf der Anwendungsphase nach 7 min beginnt das dritte Zeitintervall 12 (Regenerationsphase), wobei die Stimulationsfrequenz aus dem Theta-Frequenzband nach und nach ansteigt bis in das Beta-Frequenzband. Die Person ist nun wieder in einem aufmerksamen und konzentrierten Zustand, so dass sie ihrer gewünschten Tätigkeit nachkommen kann.

Während dieser drei Zeitintervalle 10, 11, 12 erfolgt durchgehend eine Massierung eines Rückenbereiches der Person, wobei ein entsprechendes Massagesignal 9 auf die Person einwirkt. Eine Verlaufskurve des Massagesignals 9 ist konstant über die drei Zeitintervalle 10, 11, 12.

Es versteht sich, dass die Zeitintervalle 10, 11, 12 auch variieren können. Sie können beispielsweise in einem Bereich zwischen 4 min bis 10 min betragen.

Nach einem zweiten Entspannungsprogramm B gemäß Figur 3 kann dieses dazu genutzt werden, das Einschlafen der Person zu vereinfachen. Die Ansteuerung des Gehirnstimulators 2 ist in den ersten zwei Zeitintervallen 10, 11 identisch zu der Ausführungsform nach Figur 2. Lediglich das dritte Zeitintervall 12' unterscheidet sich, wobei sich das Frequenzband in dem dritten Zeitintervall 12' - wie im zweiten Zeitintervall 11 - im Theta-Frequenzband befindet. Die Person wird somit weiterhin in den schläfrigen Zustand gehalten, wobei die Länge des dritten Zeitintervalls 12' so bemessen ist, dass nach Ablauf desselben die Person sich im Delta-Frequenzband (1 Hz- bis 3 Hz-Tiefschlaf) befindet. Das Massageansteuersignal 5 ist derart gewählt, dass das Massagesignal 9 ab dem zweiten Zeitintervall 11 linear abnimmt. Somit nimmt die Massageintensität in dem zweiten und dritten Zeitintervall 11, 12' nach und nach bzw. kontinuierlich ab.

Nach einer alternativen Ausführungsform der Erfindung können die Massageansteuersignale 5 alternativ oder zusätzlich auch diskret verlaufen unter Bildung von gleich oder unterschiedlich langen Kraftstößen als Massagesignal 9.

Vorzugsweise wird dem Stimulationssignal 7 ein Musiksignal überlagert, so dass die Person beispielsweise eine beruhigende Musik wahrnehmen kann, die der Stressbewältigung bzw. Erreichung des Entspannungszustandes förderlich ist.

Nach einer nicht dargestellten alternativen Ausführungsform der Erfindung weist die Massageeinrichtung eine Wärmefunktion auf, so dass Körperteile der Person während des Entspannungsvorganges bzw. Entspannungsprogramms gewärmt wird. Dies führt zu einer zusätzlichen Lockerung der Muskelpartien.

## Patentansprüche

1. Vorrichtung zur Erhöhung des Wohlbefindens von Lebewesen mit einem Gehirnstimulator (2) zur Erzeugung eines Stimulationssignals (7) im Infraschallbereich, mittels dessen ein Gehirn (8) des Lebewesens stimulierbar ist, mit einer Massageeinrichtung (3) zur Erzeugung eines Massagesignals (9), mittels dessen Körperpartien des Lebewesens massierbar sind, und mit einer Ansteuereinrichtung (1) zur Erzeugung eines Stimulationsansteuersignals (4) zur Ansteuerung des Gehirnstimulators (2) und eines Massageansteuersignals (5) zur Ansteuerung der Massageeinrichtung (3), wobei das Stimulationsansteuersignal (4) an den Gehirnstimulator (2) übermittelbar ist, so dass der Gehirnstimulator (2) das Stimulationssignal (7) unter Bildung einer Schwebungsfrequenz erzeugt, wobei die Schwebungsfrequenz gebildet wird als Differenz aus zwei unterschiedlichen Trägerfrequenzen im niedrigfrequenten Infraschallbereich, wobei der Gehirnstimulator (2) derart angesteuert wird, dass
- in einem ersten Zeitintervall (10) eines Entspannungsvorgangs die Schwebungsfrequenz von einer Frequenz im Bereich von 14 Hz bis 30 Hz ausgehend verringert wird,
- in einem zweiten Zeitintervall (11) die Schwebungsfrequenz einen Tiefpunkt (T) im Bereich von 3 Hz bis 7 Hz erreicht und
- in einem dritten Zeitintervall (12) die Schwebungsfrequenz auf eine Frequenz im Bereich von 14 Hz bis 30 Hz erhöht wird oder die Frequenz in dem Bereich zwischen 3 Hz bis 7 Hz beibehalten wird.

2. Vorrichtung nach Anspruch 1, **dadurch gekennzeichnet, dass** die Ansteuereinrichtung (1) als ein mobiles Datengerät ausgebildet ist, das über eine drahtlose Kommunikationsschnittstelle mit dem Gehirnstimulator (2) und der Massageeinrichtung (3) gekoppelt ist.

3. Vorrichtung nach Anspruch 2. **dadurch gekennzeichnet, dass** das mobile Datengerät als ein Mobiltelefon mit einer Anzeigeeinheit, einer Bedieneinheit und einem Mikrokontroller ausgebildet ist.

4. Vorrichtung nach einem der Ansprüche 1 bis 3, , **dadurch gekennzeichnet, dass** in der Ansteuereinrichtung (1) eine Anzahl von Entspannungsprogrammen (A, B) implementiert sind, die jeweils unterschiedliche Gehirnstimulatoransteuerungen (A1, B1) und/oder unterschiedliche Massageansteuerungen (A2, B2) aufweisen.

5. Vorrichtung nach einem der Ansprüche 1 bis 4, **dadurch gekennzeichnet, dass** dem Stimulationssignal (7) ein Musiksignal überlagert ist.

## Claims

1. A device for increasing the well-being of living beings, comprising a brain stimulator (2) adapted for generating a stimulation signal (7) in the infrasonic range, the stimulation signal (7) adapted to stimulate the brain (8) of the living being, a massager (3) adapted for generating a massage signal (9) for massaging body parts of the living being and an actuator (1) adapted for generating a stimulation actuation signal (4) for actuating the brain stimulator (2) and generating a massage actuation signal (5) for actuating the massager (3), where the said stimulation actuation signal (4) can be transmitted to the brain stimulator (2), so that the brain stimulator (2) generates the stimulation signal (7) to form a beat frequency, wherein the beat frequency is formed as the difference between two different carrier frequencies in the low-frequency infrasonic range, whereby the brain stimulator (2) can be actuated such that
- in a first time interval (10) of a relaxation process, the beat frequency of a frequency in the range from 14 Hz to 30 Hz is reduced,
- in a second time interval (11), the beat frequency reaches a lowest point (T) in the range from 3 Hz to 7 Hz, and
- in a third time interval (12), the beat frequency is increased to a frequency in the range of 14 Hz to 30 Hz or the frequency in the range between 3 Hz to 7 Hz is maintained.

2. The device according to Claim 1, **characterized in that** the actuator (1) is formed as a mobile data apparatus which is coupled to the brain stimulator (2) and the massager (3) via a wireless communication interface.

3. The device according to Claim 2, **characterized in that** the mobile data apparatus is formed as a mobile phone with a display unit, an operating unit, and a micro-controller.

4. The device according to any one of Claims 1 to 3, **characterized in that** a number of relaxation programs (A, B) are implemented in the actuator (1), each having different brain stimulation controllers (A1, B1) and/or different massage actuations (A2, B2).

5. The device according to any one of Claims 1 to 4, **characterized in that** the stimulation signal (7) is overlaid with a music signal.

## Revendications

1. Dispositif destiné à accroître le bien-être des êtres vivants, comprenant un stimulateur cérébral (2) apte à générer un signal de stimulation (7) dans la gamme des infrasons, le signal de stimulation (7) étant apte à stimuler le cerveau (8) de l'être vivant, un masseur (3) apte à générer un signal de massage (9) pour masser les parties du corps de l'être vivant et un actionneur (1) apte à générer un signal d'actionnement de stimulation (4) pour actionner le stimulateur cérébral (2) et à générer un signal d'actionnement de massage (5) pour actionner le masseur (3), le signal d'actionnement de stimulation (4) peut être transmis au stimulateur cérébral (2), de sorte que le stimulateur cérébral (2) génère le signal de stimulation (7) pour former une fréquence de battement, la fréquence de battement étant formée par la différence entre deux fréquences porteuses différentes dans la gamme des infrasons à basse fréquence, le stimulateur cérébral (2) pouvant être actionné de telle sorte que
- dans un premier intervalle de temps (10) d'un processus de relaxation, la fréquence de battement d'une fréquence comprise entre 14 Hz et 30 Hz est réduite,
- dans un deuxième intervalle de temps (11), la fréquence de battement atteigne un point le plus bas (T) dans la plage de 3 Hz à 7 Hz, et
- dans un troisième intervalle de temps (12), la fréquence de battement est augmentée jusqu'à une fréquence comprise entre 14 Hz et 30 Hz ou la fréquence comprise entre 3 Hz et 7 Hz est maintenue.

2. Le dispositif selon la revendication 1, **caractérisé par le fait que** l'actionneur (1) se présente sous la forme d'un appareil de données mobile couplé au stimulateur cérébral (2) et au masseur (3) par l'intermédiaire d'une interface de communication sans fil.

3. Dispositif selon la revendication 2, **caractérisé par le fait que** l'appareil de données mobile est constitué d'un téléphone mobile doté d'une unité d'affichage, d'une unité d'exploitation et d'un microcontrôleur.

4. Le dispositif selon l'une des revendications 1 à 3, **caractérisé par le fait qu'**un certain nombre de programmes de relaxation (A, B) sont mis en oeuvre dans l'actionneur (1), chacun ayant des contrôleurs de stimulation cérébrale différents (A1, B1) et/ou des actions de massage différentes (A2, B2).

5. L'appareil selon l'une des revendications 1 à 4, **caractérisé par le fait que** le signal de stimulation (7) est superposé à un signal musical.
